(19) Europäisches Patentamt

European Patent Office

Office européen des brevets

(11) **EP 0 801 298 B1**

(12) **EUROPEAN PATENT SPECIFICATION**

(45) Date of publication and mention
of the grant of the patent:
**21.06.2006 Bulletin 2006/25**

(51) Int Cl.:
***G01N 21/35*** *(2006.01)*

(21) Application number: **96430002.4**

(22) Date of filing: **09.04.1996**

(54) **Control of process**

Steuerung eines Prozesses

Procédé de commande de processus

(84) Designated Contracting States:
**BE DE FR GB IT NL**

(43) Date of publication of application:
**15.10.1997 Bulletin 1997/42**

(73) Proprietors:
- **INNOVENE EUROPE LIMITED
  Staines
  Middlesex,
  TW18 1DT (GB)**
- **NAPHTACHIMIE S.A.
  F-92400 Courbevoie (FR)**

(72) Inventors:
- **Lambert,Didier
  13920 Saint-Mitre Les Remparts (FR)**
- **Llinas,Jean Richard
  13008 Marseille (FR)**

- **Descales,Bernard
  13004 Marseille (FR)**
- **Martens,André
  13220 Chateauneuf les Martigues (FR)**
- **Granzotto,Claude
  13330 Pélisanne (FR)**

(74) Representative: **Preece, Michael et al
Innovene Europe Limited
European Patents Group
Compass Point
79-87 Kingston Road
Staines, Middlesex TW18 1DT (GB)**

(56) References cited:
| | |
|---|---|
| **EP-A- 0 345 182** | **EP-A- 0 437 829** |
| **EP-A- 0 607 048** | **EP-A- 0 625 702** |
| **EP-A- 0 631 810** | **EP-B- 0 801 299** |
| **US-A- 4 882 755** | **US-A- 5 023 804** |
| **US-A- 5 153 140** | **US-A- 5 361 912** |

**Description**

This invention relates to a method of controlling by near infra red (NIR) spectroscopy a steam cracking process, in particular involving hydrocarbons, especially in hydrocarbon refineries.

NIR spectroscopy has many advantages over other methods of analysis e.g. in refineries and can cover a large number of repetitive applications accurately, quickly and on line. The NIR region between 800 and 2500nm contains the totality of molecular information in the form of combinations and overtones from polyatomic vibrations, but Mathematical techniques are needed to exploit this information and to calculate the desired parameters. EP-A-285251, 304232, 305090 and 345182, the disclosure of which is hereby incorporated by reference, describe the use of NIR for determining octane number of a product, or determining yields and/or properties of a product of a chemical process in a refinery or separation process from analysis on the feeds to that process, and yields and/or properties of a product of a blending operation again from analysis on the feed thereto and for determining at least one condition of operation of a steam cracking operation. US 5 023 804 relates to a method and system for comparison of spectra. US 5 361 912 relates to an apparatus and a process for classifying the contents of non-disposable beverage bottles containers.

At present, numerical methods described for modelling physicochemical properties based on NIR spectra are all of a correlative nature and involve relations of a regressional character between the property(ies) studied. Among these multivariable analyses are multilinear regression (MLR), Principle Component Regression (PLR), Canonic regression, and regression by Partial Least Squares (PLS). In all cases there is sought between the property and the NIR spectrum a relation which may be linear but is usually quadratic or of higher algebraic form involving regression coefficients applied to each absorption. The establishment of any regression requires a progressive calibration, as the approach is empirical and not supported by a theory.

These techniques have disadvantages, the chief of which is the need for establishing a strong correlation between the spectrum and the property, and their difficulty in dealing with positive or negative synergy between components contributing to that property. Furthermore there are other practical difficulties, mainly in the need to identify samples of families having the same kind of relation between the spectra and the properties to be modelled. Thus the model may be limited especially with a non linear relation between spectrum and property. Especially when at the edges of the available data the accuracy of the model diminishes. The stability of the model is also a problem, as is the need when adding new standards to do laborious revisions to give the new model, especially when adjusting to a new feedstock for a process; thus testing the yields on 4 products leaving a steam cracker each of which has to be changed for each change of the feed not included in the calibration.

We have discovered a new approach avoiding the above problems with correlations, and regression calculations.

The present invention provides a method of controlling a steam cracking process for which a material X is a feed, in order to keep substantially constant the value $V_c$ of a property P of said feed or yield of said process, which method comprises measuring the absorption $D_i x$ of said material at more than one wavelength in the region 600-2600nm, comparing signals (i) indicative of said absorptions or mathematical functions thereof with signals (ii) indicative of absorptions $D_i m$ at the same wavelengths or mathematical functions thereof for at least 2 standards S for which the said property or yield has known value V, and at least one of said standards $S_{mc}$ having said value $V_c$ for said property or yield and controlling said process to ensure that said standard(s) $S_{mc}$ is/are the standard or standards having the smaller or smallest average values of the absolute values of the difference at each wavelength i between the signal for the material and the signal for the standard $S_m$.

The above method can be performed without regression or correlation techniques, e.g. between the absorption at any wavelength of the material and the property/yield. This method can also be performed without determining said property or yield of said process before controlling the process.

Thus for the performance of the method of the invention, a bank can be prepared in which the NIR spectra are recorded at many wavelengths for a large number of standard materials, together with their properties (or those of products obtained by processes therefrom) determined by alternative techniques e.g. gas chromatography for chemical compositions and yields determined by known methods or viscosities by known mechanical methods. The standards are chosen to cover the area in which the method is to be used, so for control of the yield of ethylene, a range of feeds to the steam cracker is chosen and under constant cracking conditions e.g. temperature pressure and catalyst yield of ethylene is measured. The number of wavelengths chosen may be 2-1000 e.g. 5-200 or 20-90 such as 60-80. The number of standards can be at least 100 or 1000, or 100,000 up to 5 million depending on property(ies) chosen.

The wavelengths chosen may be at regular intervals such as each 1-50 or 15-35nm (or each 1-5nm or each nanometre) or may be at irregular intervals e.g. with intervals of 1-200nm e.g. 1-100 or 1-50 such as 1-35nm, which may be random or chosen because of a change in the shape of the spectral curve at that wavelength e.g. a peak, trough or shoulder or chosen by chemical or statistical criteria such as factor analysis. The wavelengths may be in the region 600-2600nm, such as 1000-2600nm, in particular 1500-2600 or 2000-2550nm. The wavenumbers may be in the region 16,600-3840cm$^{-1}$, e.g. 10,000 to 4000cm$^{-1}$ in particular 6660-3840 or 5000-3900cm$^{-1}$.

Corresponding frequencies in Hertz can be obtained by multiplying these wavenumbers by $3 \times 10^{10}$ cm/sec.

The signals eg absorptions (or derivatives) for the unknown sample are compared with the signals eg absorptions (or derivatives) at the same wavelength of the standards, and those standards chosen having the smallest differences. In the method of this invention, the process is controlled so that at least one of standards chosen is one $S_{mc}$ having the desired value $V_c$ for the property or yield. The absorptions at more than one wavelength may be chosen, e.g. 2-1000 such as 5-100 or 10-20. Other methods of signal processing apart from derivatives such as Fourier transformation may be used in a similar way.

In the method of the invention the standards chosen are those with the smallest average values of the absolute difference at each wavelength i between the signal exemplified by absorption/optical density (or a derivative thereof) $D_{ix}$ for the unknown material and the corresponding signal eg absorption/optical density (or derivative thereof) $D_{im}$ for the standard. The averages may be in respect of the mean value of $D_{ix}-D_{im}$ (whatever its sign i.e. absolute difference), or $(D_{ix}-D_{im})^2$ and may be the simple mean value or the differences may be weighted to take account of the different sensitivity of the absorption to the property at that wavelength or the different sensitivity of the spectrometer at that wavelength. For each standard in the bank of standards for the type of material in question, the average difference is found as described and the standard or standards with the smallest average differences chosen, e.g. at least 1 but preferably at least 2 such as upto 1000 smallest such as 1 (or 2)-100 or 1 (or 2)-20 but is particular 1 (or 2)-10 and especially 2-6 smallest. Advantageously the average differences chosen and hence the standard (or standards) $S_m$ chosen for the property or yield wanted are such that in relation to the unknown material X and each chosen standard $S_m$ the following function is met

$$\frac{i_{xm}}{\Sigma D_{ix}} < \text{experimental error}$$

wherein $i_{xm}$ is the proximity index and is defined by $i^2(xm) = \Sigma(D_{ix}-D_{im})^2$ and the experimental error is in determining said property or yield in the standard. If more than one standard $S_{mc}$ meets the proximity index function then the average of the $S_{mc}$ values usually corresponds to the desired value $V_c$, especially the arithmetic mean, but optionally with averaging. In a modification of the method of this invention the signals (ii) are indicative of absorptions $D_{im}$ at the same wavelength or a mathematical function thereof of one standard $S_{mc}$ having the known value $V_c$ of said property or yield and controlling said process to ensure that the above function is met.

In order to aid the choice of the appropriate standards, especially in relation to a large number of wavelengths for a complex unknown mixture, it is preferred to limit the choice to those defined by means of a minimal index. For the chosen standard the minimal index is at least the same as the differences between the absorptions of the unknown and the standards. Mathematically, this may be expressed as $i^2ab \leq i^2M$ where iM is the minimal index for the property, and iab is a measure of the deviation (called the proximity index) at all the chosen wavelengths between absorption of the unknown and a chosen standard b. That measure is defined by

$$i(ab)^2 = \Sigma_i (D_{ia}-D_{ib})^2 \qquad (I)$$

where $D_{ia}$ is the optical density (or absorbence) of unknown a at wavelength i (or a derivative thereof e.g. a first, second or third derivative of that density), and $D_{ib}$ is the optical density (or absorbence) of standard b at that wavelength i (or a derivative thereof e.g. a first, second or third derivative of that density). The value of $D_1$ is the optical density or the optical density difference with respect to the baseline of the spectrum at that wavelength, or the baseline interpolated between 2 wavelengths on either side thereof. If desired signals corresponding to other mathematical functions of the absorption eg after Fourier transformation or spectral subtraction or division may be used to provide corresponding proximity and Minimal Indices.

If desired instead of the optical density $D_i$ a normalized density $W_i$ may be used where $W_i = D_i/\Sigma D_i$. This normalization avoids errors due to small electronic fluctuations in the apparatus and compensates for small differences in the optical path between the optical cells.. In this case the proximity index is defined by

$$I(ab)^2 = \Sigma_i (W_{ia}-W_{ib})^2 \qquad (2)$$

The indices can be weighted as desired for increasing resolution. One approach is to define the indices as follows.

$$I(ab)^m = \sum \text{Abs value } (X_{ia} - X_{ib})^m / \sigma_i^n \qquad (3)$$

where $X_i$ is $D_i$ or $W_i$ or a mathematical combination thereof, $\sigma_i$ is the standard deviation of X for the set of samples considered (at that wavelength) and each of m and n which are the same or different is weighting factor which is positive but can be a whole number or a fraction. Other variants can be used with other weighting factors such as those involving the spectral experimental error $e_i$, where $e_i$ is the reproducibility of the spectral measurement at wavelength i. The choice between the different options for the weighted indices may be dictated by numerical efficiency.

The reproducibility of the experimental measurements in the standards may be at least 90% or 94% or 95%. The minimal index may be obtained from a reference standard samples set according to the following procedure, hereafter called the Minimal Index Procedure. The NIR spectra for 2 standard samples A and B and their property P or yield of product are determined. By means of equation (1), (2) or (3), the value of the proximity index $i_{ab}$ is determined via the absorptions at a series of wavelengths; this index is applicable to the difference in properties $P_a-P_b$ called $EP_{ab}$.

This process is repeated with other pairs of standards c and d, e and f etc to obtain a series of Proximity Indices $i_{cd}$ etc with corresponding property differences $EP_{cd}$ etc. For different values of a parameter L which is greater than the indices $i_{ab}$ etc, the corresponding values of $EP_{ab}$ etc are averaged to give an average $EP_{ij}$ for that value of L; the different values of $EP_{ij}+t\sigma/\sqrt{K}$ are then plotted on a graph against L $\sigma$ is the accuracy of the property determination and K is the number of pairs of samples for which $i_{ab}$ is inferior to a given L. t is the Student factor at a given level of confidence. The intercept is then measured between the curve obtained and a line usually horizontal which is the reproducibility of the property level at an appropriate confidence interval e.g. 90% or more usually 95%; the abcissa portion of the intercept gives the minimal index $i_{min}$, which is the minimum value of $i_{ab}$ for which $P_a$=Pb within the frame of experimental error.

From this minimal index by Procedure 1, the standards can be chosen which have values of $i^2_{ab} \leq i^2_{min}$ where in this case a is the unknown and b is a standard, as in this case the difference between Property or yield a and Property or yield b is less than or equal to $\sigma\sqrt{2}$, where $\sigma$ is the experimental error in measuring the property or yield. Then if the standard meeting this requirement is $S_{mc}$ with property or yield value $V_c$, the process is under control, but if a different standard is nearest to the unknown then the process needs adjustment as described below.

The process may be controlled to keep substantially constant more than one Property or yield at once, e.g. at least 2, such as 1-30 e.g. 2-10 properties or yields at once. Each property of the standards has a particular unweighted, minimal index, which may lie in the region $0-10^{-10}$ e.g. $10^{-2}$ to $10^{-8}$, in particular $10^{-5}$ to $10^{-8}$ in particular $10^{-8}$ to 5X, $10^{-7}$ or $5\times10^{-7}$ to $5\times10^{-4}$ for Minimal Indices derived from absorbancies; corresponding Minimal Indices may be obtained for other signals/functions. If the Minimal Index chosen is the smallest for all the properties or yields desired, then the same one may be used for all the properties or yields and the standards chosen will be suitable for all the properties or yields. The Minimal Index for each property or yield may be used separately, with different numbers of standards chosen for each property or yield (assuming different Minimal Indices). If desired the same Minimal Index may be used, which is not the smallest, resulting in some of the chosen standards (with a higher Minimal Index) giving some properties or yields of high accuracy and some (with a lower Minimal Index) giving some properties or yields of less high accuracy.

The value of the property or yield to be controlled may be of the feed sample being analyzed or yield of a product obtained from that sample as the value obtained is derived from the standards, and they will have been determined as needed for the eventual use. EP304232, 305090 and 345182 referred to above describes such techniques when applied to use of NIR with correlation to blending, separating or cracking operation; the same principles apply in the present method.

If the density of the standards in the data bank is sufficient to have $i^2ab \leq i^2$ min as is usually the case, the above procedure is very satisfactory. But there are occasions when the bank is incomplete, because of shortage of data of properties in a particular area i.e. a low density of standards or the sensitivity of the property to changes in absorption is so small, that a very small Minimal Index is required and there may be few standards with proximity indices meeting it. It is possible simply choose a larger Minimal Index with e.g. 1-5 times such as 1.5-2 times the Minimal Index; the results may be less accurate than those from a smaller minimal index.

However, a more accurate approach with a low density of standards involves a special densification process of Procedure 2, in which random or semi random densification of the neighbourhood of the unknown is achieved by generation of synthetic standards, based on standards already in the bank. Each new synthetic standard may be obtained from combinations of standards taken at random from the bank but preferably it is obtained from the other standards by the constraint of choosing only a mixture of N standards for which

$$(\text{Min})C_j - u_j \leq C_{ij} \leq (\text{Max})C_j + u_j \qquad (4)$$

and

$$\sum C_{ij} = 1 \qquad (5)$$

where $C_{ij}$ is the fraction of component j in the sample $_i$.

Min $C_j$ is the minimum amount of $_j$ in the initial calibration mixture i.e. standards in the bank or in the samples for which the method is to be used, and

Max $C_j$ is the maximum amount of $_j$ in the initial calibration mixture i.e. standards in the bank or in the samples for which the method is to be used, and

uj is usually between 1 and 0.01 or 1 and 0.05 preferably between 0.5 and 0.1 and can be fixed for each property or yield.

The constraints over the choice of such mixtures of N standards can also be equally fixed in the spectral area from which the samples will be drawn in order to remain in the areas of similar chemical nature.

The number of samples effectively drawn into the bank in this densification can be of several thousand generally 1000-2000. The calculation time is extended without significant deterioration in the results. If no further neighbours are found, the trawl of new samples drawn in is enlarged.

The spectrum of each mixture is calculated by the combination of the spectra of the standards used according to the formula

$$S_{Mi} = \sum C_{ij} X S_j \qquad (6)$$

where $S_j$ is the spectrum in the mixture of component $_j$ in the calibration matrix.

The properties or yields of each mixture PMi can be calculated by a generally linear combination of the properties or yields of the standards according to the formula

$$P_{Mi} = \sum C_{ij} X P_j \qquad (7)$$

where Pj is the property of component j

In the case of non linear additive properties, appropriate mixing factors can be applied.

Having obtained the spectrum and the properties or yields of the synthetic mixtures, these can be used as "standards" to help control the process by keeping the properties or yields constant in the same way as a conventional standard.

Instead of using either of the two above approaches, 1-7, a third type Procedure 3 may be used as follows. The Q nearest samples to unknown X can be found from a selection from the bank samples for which the proximity index to the unknown sample is (V) X $i_{min}$) where v is 0.1 < v < 10, (8) preferably 0.5<v<2 or $1 \le v \le 5$. Then by the method of least squares is found a generally linear combination of the standard products, which are the Q nearest samples, to reproduce the spectrum of X according to the equation.

$$S_X = \sum C_R X S_r \qquad (9)$$

where $C_r$ is the coefficient for sample R in the total Q and $S_R$ is the spectrum of sample R. The coefficient $C_R$ which can be normalized to $C_R = 1$ or not and/or optimized by the least squares route, allows an estimation of the property $P_x$ according to the equation.

$$P_X = \sum C_R X P_R \qquad (10)$$

where $P_R$ is the property of sample R.

The eventual size of the estimation error can be derived by application of Gaussian theory, also called the propagation error (see Eq.10).

The above third approach can only be applied if the product X is situated inside the maximum extension of the standard products defined by equation (8) i.e. within the range of bank samples defined in equation (8). If this is not the case, X is outside the field of the actual bank of products and escapes from the area of knowledge of the method into the area of learning.

The densification process described in relation to equations 4-7, or 9 or 10 is usually applied to the method of the invention involving no correlation or regression techniques. However, if desired the densification process may be applied to increase the number of "standards" for consideration in an NIR analytical technique involving the correlation on regression techniques as described above e.g. MLR. This method provides for adding an extra synthetic standard to a bank of known standards, each of which relates at least one absorption in the 600-2600nm region (or a signal indicative thereof or of a mathematical function of said absorption eg a derivative thereof) of a feed to a steam cracking process to a known property of that feed or yield of said process, which method comprises choosing from the bank at least 2 standards for which equations 4 and 5 above are met, considering mixing the chosen standards in at least one proportion to produce at least one mixture for use as a synthetic standard, and estimating the spectrum and property/ yield of said mixture according to equation 6 and 7 respectively.

The spectrum and property/yield of each "mixture" can then be added to the bank and used to develop models through the known correlation/regression approach, e.g. as described EP345182.

As explained above if the nearest standard to the unknown is not one having the value $V_c$ for the property or yield, or having a value V for said property or yield within $\pm$ 10%, e.g. $\pm$ 5% or $\pm$ 1% of said value $V_c$, or if the function $i_{xm}/\Sigma D_{ix}$ is greater than the experimental error, especially more than 10%, 5% or 1% greater, then the process has deviated and needs adjustment, e.g. by changing one of the parameters of the process e.g. reaction conditions such as temperature, pressure, or amount/nature of catalyst, or proportions or nature of the feeds.

The method of this invention is applicable to control of the properties of feeds to a steam cracking, as well as the determination from the feed to the steam cracker of the potential yields of $C_2$-$C_6$ alkenes e.g. ethylene, propylene, butenes and a $C_5$ olefin fraction, or benzene in the steam cracking under standard conditions. Other "properties" of the feed which relate to its suitability for use in steam cracking under standard conditions may be measured such as its potential yield of methane and its cokability, ie the tendency to form coke in that cracking. Other properties of the feed that can be determined in addition include its chemical composition eg percentages of linear paraffinic hydrocarbons, isoparaffins, naphthenics compounds, aromatics and benzene, and its density and mean molecular weight. These parameters are important for the smooth operation of a steam cracker, as variations affect that operation, especially the olefin production. Such variations may result from changes intentional or otherwise in the feeds, which may be naphtha, condensates, liquefied gas and/or gas oil. The steam cracking may involve amounts of steam of 30-50% eg 40% (relative to hydrocarbon feed) riser inlet temperatures of 100-400°C eg 250°C and riser outlet temperature of 800-900°C eg 830°C and residence time of 0.1-5 sec eg 0.5 sec.

In each case the method may be applied to determine the nearest standards of property or yield and then the process controlled e.g. on line and especially with continous feed back from the results to control the production process.

In each of the above processes the control may be performed and notice taken of any deviations by adjusting the parameters of the cracking process e.g. flow rates proportion or nature of feed(s) i.e. steam or hydrocarbon (e.g. via operation of control valves) and/or temperature/pressure etc to bring the property or yield back to the desired figure. This control of the cracking process, is usually performed with a micro computer which is linked to the spectrometer and also performs the search for the standards Sm. The inline control of the process is very efficient and very fast.

The present invention also provides an apparatus suitable for carrying out the method of the invention comprising an infra red spectrometer and a computer wherein the infra red spectrometer is linked to the computer programmed in such manner to determine the nearest standard, and this in turn is linked to a control means to adjust the process in relation to any deviations when $S_{mc}$ is not the nearest standard. The spectrometer is suitable for measuring spectra in at least partly in the 600-2600nm wavelength range and can be linked to a signal processing device to allow numerical treatment of the spectrum, preferably by Fourier Transformation. The spectrometer receives at least one signal from a vessel containing product or from a feed or product line. The information obtained can be used as an information vector for the computer which is programmed to determine the nearest standard e.g. via calculations on the proximity indices in relation to standards. Conveniently in relation to the process, the computer may be used in a closed loop feed back or feed forward control system for controlling processing equipment e.g. changing the process parameters in response to variations in the nearest standard from measurement of more than one absorptions in the NIR spectrum of the feed.

The present invention also provides a computer programmed to perform the method of the invention. The apparatus for use with the former method of the invention comprises an NIR spectrometer receiving at least one signal from a feed line in said process and being coupled to a computer to effect continuous measurement of the spectra of the feed and provide feed back or feed forward control of the process. The present invention also provides a computer implemented method for a system including a spectrometer linked to a process line containing a material X, which is a feed to a steam cracking process a computer linked to the spectrometer, and a controller linked to the computer and the process line, the computer including databanks having stored therein signals indicative of absorptions of standard materials (or mathematical functions thereof) and corresponding properties of said materials, or yields of said process, the method comprises steps of:

measuring absorption at more than one wavelength in the region 600-2600nm at the process line and producing absorption signals (or mathematical functions eg derivatives thereof) by the spectrometer in accordance therewith;
accessing the databanks of the computer in accordance with the absorption signals (or functions thereof);
comparing, by the computer, the absorption signals (or functions thereof) to the signals (or functions thereof) of the standard materials stored in the databanks;
choosing at least one standard based on the comparing, said standard having the smallest average value of the absolute difference at each wavelength i between the signal for the absorption (or function thereof) for the material and the signal (or function thereof) for the standards; and
controlling said process directly in accordance with the outputted standard, to ensure standard $S_{mc}$ is the one with the smallest average value.

The benefits of invention allow improvements in control of processes involving modelling with the following areas, identification and classification of novel feeds, simultaneous control of all of the properties on a sample without the need for generating different models for each. The method of the invention overcomes the difficulties with the classical regressional approach, in particular avoiding all difficulties with numerical stability of the models.

The method also allows an extension of the field of application of the method without the need to rewrite the model, apart from the need to integrate the new samples which are inside or outside the previous field of validity of the method. This possibility of automatic learning, which is not possessed by traditional regression techniques, is a decisive advantage in the framework of continuous inline industrial control processes, because it allows the return of the industrial plant operations to the model in a certain and rapid manner in a minimum time and with all the properties considered in the model. In contrast classical regression methods would necessitate the redevelopment of all the models, which is long and laborious without being able to guarantee the result of the new model obtained, because a new validation period is necessary; in addition during the redevelopment of the model any commercial use e.g. in a refinery of the model is very limited. Furthermore, the method of invention allows equally the easy extension to a number of properties or yields, which are simply incorporated into the known bank.

This remarkable possibility is true not only for control of processes with conventional properties such as physical chemical and/or rheological properties, but also for complex ones. The methods of the invention equally allow application of the models from one apparatus to another and from one spectral region to another, where conventional regressive method cannot give satisfactory solutions. This apparatus portability is made possible by the fact that the differences between different spectra are the same in one apparatus as another, for the same type of spectrometer being considered (e.g. network scatter, Fourier transform, accousto optical system AOTS, diode array etc). This portability between spectral regions depends on the fact that as the spectral regions are intercorrelated, the relations between the spectra are maintained between one another.

The invention is illustrated in the accompanying Figures in which :

Figure 1 represents a schematic diagram showing apparatus for use in the invention;
Figure 2 represents a schematic block flow diagram for the method of the invention.

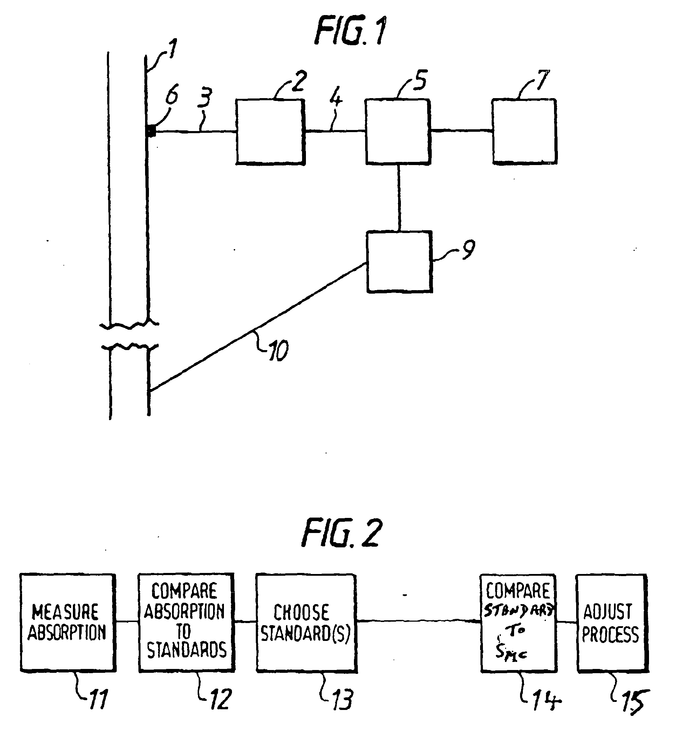

In Figure 1, an optical fibre 3 links a spectrometer 2 and a probe 6 in or at process line 1. The spectrophotometer 2 produces absorbance signals at more than 1 wavelength, which signals as such (or after mathematical treatment to form e.g. derivative signals) are passed via line 4 to computer 5, where the signals as such or after conversion e.g. to one or more derivative signals, are used to enable the computer to access the databank 7 of standard signals eg absorptions and properties/yields therein. The signals are compared to those of one or more standard absorption(s) as described above. The output of the computer 5 is in the form of a signal which is used to control the process involved with the product in line 1, ie for which line 1 is a feed line; in this case the computer 5 is linked to and instructs the controller 9 which, via 10, controls that process eg. via valves/temperature and/or pressure controls in line 1 or in relation to line 1. By this means the property of material in line 1 or yield of product of the process from that material can be kept substantially constant without the need to determine that property or yield.

In Figure 2, the initial operation 11 is to measure the absorption of the unknown, after which in the second step 12, the absorptions are compared to absorptions in spectra of standards, and in the third step 13, the spectra of the standards Sm are chosen according to criteria described above, and then in step 14, if the standard $S_m$ chosen is not $S_{mc}$, in step 15 the process involving the unknown is adjusted to keep the standard chosen to be $S_{mc}$ and hence to keep the value of the property or yield substantially constant.

The invention is illustrated in the following Examples in which the Minimal Index is calculated according to the Minimal Index Procedure described above. Mathematically the steps concerned are as follows.

For each couple of standard samples i, j, the Proximity Index iij is determined from the NIR spectra by use of equation 1, 2, or 3 and the properties are measured. For each Proximity Index is calculated the absolute difference $EP_{ij}$ between the properties of the samples. The Minimal Index for property or yield P is obtained from the average $(EM_pL)$ of $EP_{ij}$ for different values of L when $L \geq ij$. Thus the $EM_p(L) = 1/K \, \Sigma_i \, \Sigma_j \, EP_{ij}$ for each of K samples for which $i_{ij} \leq L$.

$EMp(L)+t\sigma(M)$ is plotted against the proximity index and in addition there is plotted the reproducibility of the standard method at a given level of confidence, as defined in the Minimal Index Procedure above. The intercept of the curve from EMpL and the reproducibility give the upper limit i.e. the Minimal Index.

For the Examples the data is expressed in Tables in a form as shown below in the data is as follows:

| | | Absorption | | | | |
|---|---|---|---|---|---|---|
| | | Weighting | Unknown | Estimated | Standard A | Standard B |
| Proximity Index | | | | | | |
| Wavelength λ | | | | | | |
| cm$^{-1}$ | nm | | | | | |
| | | | | | | |
| | | | | | | |
| | | | | | | |
| Property l | | | | | | |
| Property j | | | | | | |
| Property m | | | | | | |

The wavelengths chosen are shown in columns 1 and 2.

Column 3 gives the weight loading associated with each wavelength for the proximity index for the standards; 1 denotes no loading.

Column 4 shows for the unknown sample the absorption at the various wavelengths and at the bottom the properties of that sample determined by standard methods.

Column 5 shows for the unknown sample the estimated values of the properties and the absorptions based on the properties and absorptions of the chosen standards.

Columns 6, 7 etc show the values of the absorptions and properties for the standards chosen from the bank.

Line 2 give the value of the proximity index between the unknown sample and each of the chosen standards.

Example 1

Control of a steam cracking.

A mixture of hydrocarbons, a naphtha, a petroleum hydrocarbon cut, was a feed to a steam thermal cracker for producing olefins, such as ethylene, propylene and butenes, as well as cracked gasoline. The key properties of the feed are its density and its "potential content of ethylene", called CPC2, (ie yield of ethylene product) which is its capacity to product ethylene under standard steam cracking conditions. In addition, other important properties are its cokability Index (1C), which is the potential of the feed to generate coke or its yields of coke [under the standard conditions of steam cracking] as well as the chemical composition eg the percentages of linear paraffinic hydrocarbon (LIN%), isoparaffins ISO%), naphthenics (NAPHT%) aromatics (ARO%) and benzene (BENZ%).

The NIR spectrum of a naphtha F on line to a steam cracker furnace was measured with a Fourier Transform spectrometer of 4800-4000cm$^{-1}$ (with baseline at 4720cm$^{-1}$); the absorbances were normalized, but not weighted. The conditions of the steam cracking were % steam 40%, outlet temperature 830°C, residence time 0.5 sec. A bank of data on known naphthas with their NIR spectra and properties had been previously prepared, the properties being determined with respect to a steam cracking under the same standard conditions. By the Minimal Index Procedure and non weighting of the absorbances and Equation 2 the Minimal Index was determined as 3.7 x 10$^{-7}$. By the method of this invention the proximity indices between the unknown naphtha 1F and the bank of known naphthas were determined, and those known naphthas with proximity indices less than 3.7 x 10$^{-7}$ chosen. Five such naphthas were found, designated 1A-1E. The steam cracking process was controlled to keep the 5 standards the ones with the nearest proximity indices to the feed naphtha. Adjustments to the composition of the feed were made in case of any variations in the nearest proximity indices. The control was checked by determining by averaging from the standard naphthas 1A-1E the properties of naphtha 1F. The Table 1.1 shows the absorbances for the napthas 1A-1F, as well as the estimated absorbances for 1F and the properties of 1A-1F, and the estimated properties of 1F. The table shows the accuracy of the method for the calculated properties, which are in agreement with the standard methods, any differences between the measured and estimated properties (columns 3 and 4) being within the limits of reproducibility of the reference methods.

Other properties of the naphtha, such as average molecular weight, may be obtained in a similar way.

**TABLE 1.1 Determination of the properties of a mixture of hydrocarbons for feeding to a steam cracker**

| | | 1F measured | 1F estimated | 1A | 1B | 1C | 1D | 1E |
|---|---|---|---|---|---|---|---|---|
| | Proximity Index | | 3,80E-08 | 8,94E-08 | 9,30E-08 | 1,87E-07 | 2,84E-07 | 3,57E-07 |
| Wavelengths | | | | | | | | |
| λ (cm-1) | λ (nm) | | | | | | | |
| 4739 | 2110 | 0,000117 | 0,000114 | 0,000115 | 0,000111 | 0,000118 | 0,00011 | 0,000116 |
| 4726 | 2116 | 0,000146 | 0,0001466 | 0,000147 | 0,000142 | 0,000152 | 0,00014 | 0,000152 |
| 4717 | 2120 | 0,000185 | 0,0001866 | 0,000189 | 0,000178 | 0,000192 | 0,00018 | 0,000194 |
| 4673 | 2140 | 0,00066 | 0,0006506 | 0,00066 | 0,000628 | 0,000639 | 0,000605 | 0,000721 |
| 4669 | 2142 | 0,000677 | 0,000663 | 0,00067 | 0,00064 | 0,000653 | 0,000617 | 0,000735 |
| 4660 | 2146 | 0,000718 | 0,0007042 | 0,000711 | 0,000682 | 0,000691 | 0,00066 | 0,000777 |
| 4651 | 2150 | 0,000781 | 0,0007698 | 0,000775 | 0,000748 | 0,000756 | 0,000724 | 0,000846 |
| 4647 | 2152 | 0,000823 | 0,000811 | 0,000814 | 0,00079 | 0,000798 | 0,000764 | 0,000889 |
| 4643 | 2154 | 0,000868 | 0,000854 | 0,000859 | 0,000829 | 0,000843 | 0,000802 | 0,000937 |
| 4625 | 2162 | 0,00108 | 0,0010548 | 0,001055 | 0,001022 | 0,001043 | 0,00099 | 0,001164 |
| 4621 | 2164 | 0,001112 | 0,0010928 | 0,001092 | 0,00106 | 0,00108 | 0,001026 | 0,001206 |
| 4613 | 2168 | 0,001145 | 0,0011332 | 0,001131 | 0,001101 | 0,001117 | 0,001072 | 0,001245 |
| 4604 | 2172 | 0,001184 | 0,001175 | 0,001177 | 0,001146 | 0,001155 | 0,001123 | 0,001274 |
| 4591 | 2178 | 0,001288 | 0,0012826 | 0,001289 | 0,001254 | 0,001269 | 0,001242 | 0,001359 |
| 4583 | 2182 | 0,001376 | 0,001366 | 0,00137 | 0,001343 | 0,001357 | 0,001325 | 0,001435 |
| 4550 | 2198 | 0,001962 | 0,0019714 | 0,001965 | 0,001952 | 0,001974 | 0,001951 | 0,002015 |
| 4537 | 2204 | 0,00223 | 0,002231 | 0,002223 | 0,002208 | 0,002235 | 0,002213 | 0,002276 |
| 4529 | 2208 | 0,002364 | 0,0023672 | 0,00236 | 0,002344 | 0,002372 | 0,00235 | 0,00241 |
| 4496 | 2224 | 0,002767 | 0,0027762 | 0,002765 | 0,002749 | 0,002777 | 0,002762 | 0,002828 |
| 4484 | 2230 | 0,003013 | 0,0030192 | 0,003005 | 0,002996 | 0,003026 | 0,003004 | 0,003065 |
| 4472 | 2236 | 0,003372 | 0,0033802 | 0,00337 | 0,003356 | 0,003385 | 0,003362 | 0,003428 |
| 4405 | 2270 | 0,015722 | 0,0157656 | 0,015732 | 0,015737 | 0,015831 | 0,015774 | 0,015754 |
| 4394 | 2276 | 0,017032 | 0,0170668 | 0,01706 | 0,017053 | 0,01707 | 0,0171 | 0,017051 |
| 4390 | 2278 | 0,017074 | 0,0171032 | 0,017113 | 0,017089 | 0,017089 | 0,017146 | 0,017079 |
| 4386 | 2280 | 0,017026 | 0,0170488 | 0,017072 | 0,017032 | 0,017035 | 0,017082 | 0,017023 |
| 4382 | 2282 | 0,016925 | 0,0169442 | 0,016977 | 0,01692 | 0,01692 | 0,016989 | 0,016915 |
| 4378 | 2284 | 0,016825 | 0,0168448 | 0,016882 | 0,016823 | 0,016815 | 0,016888 | 0,016816 |
| 4374 | 2286 | 0,016793 | 0,0168204 | 0,016862 | 0,016796 | 0,016788 | 0,016865 | 0,016791 |

Table continued

| | | 1F measured | 1F estimated | 1A | 1B | 1C | 1D | 1E |
|---|---|---|---|---|---|---|---|---|
| 4367 | 2290 | 0,017293 | 0,0173046 | 0,017329 | 0,017281 | 0,017284 | 0,017349 | 0,01728 |
| 4340 | 2304 | 0,026754 | 0,0267546 | 0,026706 | 0,026739 | 0,026904 | 0,026781 | 0,026643 |
| 4337 | 2306 | 0,027159 | 0,0272098 | 0,027144 | 0,027223 | 0,027335 | 0,027238 | 0,027109 |
| 4333 | 2308 | 0,026844 | 0,026879 | 0,026816 | 0,026912 | 0,026963 | 0,026899 | 0,026805 |
| 4329 | 2310 | 0,025944 | 0,0259448 | 0,025889 | 0,026002 | 0,025989 | 0,025933 | 0,025911 |
| 4318 | 2316 | 0,022338 | 0,0223474 | 0,022282 | 0,022401 | 0,022344 | 0,022343 | 0,022367 |
| 4303 | 2324 | 0,019545 | 0,0195754 | 0,019539 | 0,019589 | 0,019601 | 0,019574 | 0,019574 |
| 4292 | 2330 | 0,019333 | 0,0193696 | 0,019369 | 0,019367 | 0,019386 | 0,019408 | 0,019318 |
| 4281 | 2336 | 0,020823 | 0,0208396 | 0,020873 | 0,020841 | 0,02084 | 0,020909 | 0,020735 |
| 4274 | 2340 | 0,021993 | 0,0219922 | 0,022033 | 0,021986 | 0,02199 | 0,022055 | 0,021897 |
| 4270 | 2342 | 0,022316 | 0,0223228 | 0,022356 | 0,022326 | 0,022333 | 0,022367 | 0,022232 |
| 4266 | 2344 | 0,02233 | 0,0223342 | 0,022362 | 0,022345 | 0,022331 | 0,022375 | 0,022258 |
| 4263 | 2346 | 0,021983 | 0,0219904 | 0,021993 | 0,02201 | 0,021979 | 0,022023 | 0,021947 |
| 4259 | 2348 | 0,021309 | 0,0213088 | 0,021298 | 0,021331 | 0,021302 | 0,021335 | 0,021278 |
| 4255 | 2350 | 0,020391 | 0,0203998 | 0,020371 | 0,020434 | 0,020375 | 0,020412 | 0,020407 |
| 4244 | 2356 | 0,017684 | 0,0176596 | 0,017627 | 0,017695 | 0,017637 | 0,017653 | 0,017686 |
| 4219 | 2370 | 0,015586 | 0,01558 | 0,01553 | 0,015607 | 0,015596 | 0,015609 | 0,015558 |
| 4212 | 2374 | 0,015581 | 0,0155792 | 0,015539 | 0,015612 | 0,015594 | 0,015601 | 0,01555 |
| 4191 | 2386 | 0,015644 | 0,0156246 | 0,01561 | 0,015661 | 0,015633 | 0,01566 | 0,015559 |
| 4181 | 2392 | 0,016231 | 0,0162086 | 0,016218 | 0,01624 | 0,016194 | 0,016263 | 0,016128 |
| 4174 | 2396 | 0,016428 | 0,016389 | 0,016395 | 0,016417 | 0,016355 | 0,016444 | 0,016334 |
| 4170 | 2398 | 0,016332 | 0,0162994 | 0,016317 | 0,016326 | 0,01625 | 0,016354 | 0,01625 |
| 4167 | 2400 | 0,016151 | 0,016113 | 0,016129 | 0,016135 | 0,016058 | 0,016171 | 0,016072 |
| 4160 | 2404 | 0,015691 | 0,0156668 | 0,015667 | 0,01569 | 0,015628 | 0,015714 | 0,015635 |
| 4149 | 2410 | 0,015467 | 0,0154834 | 0,015461 | 0,015505 | 0,015478 | 0,015525 | 0,015448 |
| 4139 | 2416 | 0,015865 | 0,0158868 | 0,015872 | 0,015906 | 0,015899 | 0,01593 | 0,015827 |
| 4136 | 2418 | 0,016018 | 0,0160316 | 0,016016 | 0,016058 | 0,016049 | 0,016079 | 0,015956 |
| 4132 | 2420 | 0,016145 | 0,0161568 | 0,016147 | 0,016185 | 0,016166 | 0,016203 | 0,016083 |
| 4122 | 2426 | 0,016501 | 0,0165196 | 0,016531 | 0,016545 | 0,016523 | 0,016578 | 0,016421 |
| 4115 | 2430 | 0,016816 | 0,0168624 | 0,016898 | 0,016889 | 0,016876 | 0,016929 | 0,01672 |
| 4108 | 2434 | 0,01718 | 0,017225 | 0,01725 | 0,017252 | 0,017266 | 0,017278 | 0,017079 |
| 4105 | 2436 | 0,017345 | 0,0173724 | 0,017392 | 0,017409 | 0,017418 | 0,017412 | 0,017231 |
| 4102 | 2438 | 0,017449 | 0,01746 | 0,017503 | 0,017488 | 0,0175 | 0,017497 | 0,017312 |
| 4098 | 2440 | 0,017523 | 0,0175392 | 0,017563 | 0,017558 | 0,017579 | 0,017573 | 0,017423 |
| 4088 | 2446 | 0,01793 | 0,0178916 | 0,017915 | 0,017889 | 0,017922 | 0,01789 | 0,017842 |
| 4082 | 2450 | 0,018463 | 0,0184254 | 0,018443 | 0,018409 | 0,018448 | 0,0184 | 0,018427 |
| 4072 | 2456 | 0,019168 | 0,0191398 | 0,019162 | 0,019125 | 0,019123 | 0,019089 | 0,0192 |

Table continued

| | | 1F measured | 1F estimated | 1A | 1B | 1C | 1D | 1E |
|---|---|---|---|---|---|---|---|---|
| 4068 | 2458 | 0,019128 | 0,0190918 | 0,019147 | 0,019056 | 0,019049 | 0,019031 | 0,019176 |
| 4065 | 2460 | 0,018841 | 0,0188114 | 0,018879 | 0,018795 | 0,018753 | 0,018721 | 0,018909 |
| 4062 | 2462 | 0,01833 | 0,0183078 | 0,018377 | 0,018289 | 0,018246 | 0,018229 | 0,018398 |
| 4055 | 2466 | 0,016845 | 0,0168544 | 0,016868 | 0,016849 | 0,016804 | 0,016782 | 0,016969 |
| 4049 | 2470 | 0,015392 | 0,0153728 | 0,015367 | 0,015354 | 0,015347 | 0,015254 | 0,015542 |
| 4042 | 2474 | 0,014236 | 0,014211 | 0,014171 | 0,014203 | 0,014196 | 0,014095 | 0,01439 |
| 4032 | 2480 | 0,012938 | 0,0129096 | 0,012872 | 0,012905 | 0,012903 | 0,012826 | 0,013042 |
| 4026 | 2484 | 0,012416 | 0,0123958 | 0,012353 | 0,0124 | 0,012387 | 0,012354 | 0,012485 |
| 4016 | 2490 | 0,011841 | 0,0118108 | 0,011786 | 0,011821 | 0,011801 | 0,011787 | 0,011859 |
| 4010 | 2494 | 0,011216 | 0,011202 | 0,011196 | 0,011209 | 0,011179 | 0,011198 | 0,011228 |
| | | | | | | | | |
| | Density | 0,7088 | 0,7081 | 0,7084 | 0,7081 | 0,7045 | 0,7071 | 0,7124 |
| | CPC2 | 20,16 | 20,27 | 20,05 | 20,16 | 20,76 | 20,67 | 19,71 |
| | LIN% | 34,37 | 35,15 | 34,33 | 34,62 | 36,42 | 36,7 | 33,69 |
| | ISO% | 32,69 | 32,51 | 31,36 | 32,54 | 33,29 | 30,73 | 34,64 |
| | NAPHT % | 25,69 | 25,00 | 26,86 | 25,66 | 23,79 | 26,21 | 22,49 |
| | ARO% | 7,25 | 7,33 | 7,46 | 7,18 | 6,5 | 6,35 | 9,18 |
| | IC | 108,8 | 107,9 | 109,7 | 109,2 | 105,1 | 105,3 | 110,3 |
| | BENZ% | 1,05 | 1,06 | 1,22 | 1,06 | 0,93 | 1,06 | 1,07 |

In the Table 3.8 E-08 means 3.8 x $10^{-8}$

**Claims**

1.  Method of controlling a steam cracking process for which a material X is a feed in order to keep constant the value $V_c$ of a property P of said feed, or the yield of said process, which method comprises measuring the absorption $D_{ix}$ of said material at more than one wavelength in the region 600-2600nm, **characterised in that** the method further comprises comparing signals (i) indicative of said absorptions or a mathematical function thereof with signals (ii) indicative of absorptions $D_{im}$ at the same wavelengths or a mathematical function thereof for at least 2 standards $S_m$ for which the said property or yield has a known value V, at least one of said standards $S_{mc}$ having a value $V_c$ for said property or yield and controlling said process to ensure that said standard $S_{mc}$ or standard(s) $S_{mc}$ is the standard or standards having the smaller or smallest average value of the absolute difference at each wavelength i between the signal for said material and the signal from the standard $S_m$.

2.  A method according to claim 1 which comprises comparing signals (i) from said material with signals (ii) from standards $S_m$, at least 2 of which have smallest average values of the differences, and the average of the values V of the property or yield of said at least 2 standards being $V_c$.

3.  A method according to claim 1 or 2 comprising comparing absorptions $D_ix$ (or a derivative thereof) with absorption $D_im$ or a derivative thereof.

4.  A method according to any one of claims 1-3 wherein the standard $S_{mc}$ is such that in relation to the material X and the or each standard $S_{mc}$ the following function is met

$$\frac{i_{xm}}{\Sigma D_{ix}} < \text{experimental error}$$

wherein $i_{xm}$ is the proximity index and is defined by $i^2(xm) = \Sigma(D_{ix}-D_{im})^2$ and the experimental error is in determining said property or yield in the standard, and in the case where more than one standard $S_{mc}$ meets the function, averaging the properties or yields from said standards gives a value $V_c$.

5. A method according to claim 4 wherein the comparison of signals (i) is with signals (ii) indicative of absorptions $D_{im}$ at the same wavelength or a mathematical function thereof of one standard $S_{mc}$ having the known value $V_c$ of said property or yield and controlling said process to ensure that the function specified in claim 4 is met.

6. A method according to claim 4 or 5 wherein the proximity index is less than the minimal index $i_m$ which has been determined from the standards Sa, Sb, Sc ...... by (a) calculating for each pair of standards Sa/Sb, Sa/Sc the value of $i^2(a,b)$ etc, (b) relating the values of $i^2(a,b)$ etc to the corresponding differences EP (ab) in properties Pa, Pb etc (c) calculating for each value L for which $i^2(ab)$ is $\leq L$, the average of the corresponding differences EPab, (d) calculating Minimal index from the value of minimal index $i^2(ab)$ where average EPab is the same as reproducibility standard for the property.

7. A method according to any one of the preceding claims wherein the process is controlled in a closed loop control system to control the processing equipment in relation to a process for which the material X is a feed.

8. A method according to any one of claims 1-7 which comprises determining from the absorptions of the feed to said process, the yield of at least one product from the process.

9. A method according to claim 8 which comprises determining the yield of at least one of alkene 2-4 carbons, methane and coke (as expressed by a cokability index).

10. A method according to any one of the preceding claims which is computer implemented.

**Patentansprüche**

1. Verfahren zum Steuern eines Dampfcrackingverfahrens, für das ein Material X ein Zuführungsmaterial darstellt, um den Wert $V_c$ einer Eigenschaft P des Zuführungsmaterials oder die Ausbeute des Verfahrens konstant zu halten, wobei das Verfahren Messen der Absorption $D_{ix}$ des Materials bei mehr als einer Wellenlänge im Bereich von 600-2600 nm umfasst, **dadurch gekennzeichnet, dass** das Verfahren weiterhin Vergleichen von Signalen (i), die für die Absorptionen oder eine mathematische Funktion davon bezeichnend sind, mit Signalen (ii), die für Absorptionen $D_{im}$ bei den gleichen Wellenlängen oder eine mathematische Funktion davon bezeichnend sind, für mindestens 2 Standards $S_m$, für die die Eigenschaft oder Ausbeute einen bekannten Wert V aufweist, wobei mindestens einer der Standards $S_{mc}$ einen Wert $V_c$ für die Eigenschaft oder Ausbeute aufweist, und Steuern des Verfahrens, um zu sichern, dass der Standard $S_{mc}$ oder Standard(s) $S_{mc}$ den Standard oder die Standards mit dem kleineren oder kleinsten Mittelwert der absoluten Differenz bei jeder Wellenlänge i zwischen dem Signal für das Material und dem Signal von dem Standard $S_m$ darstellt.

2. Verfahren nach Anspruch 1, das Vergleichen von Signalen (i) von dem Material mit Signalen (ii) von Standards $S_m$ umfasst, wobei mindestens 2 davon kleinste mittlere Werte der Unterschiede aufweisen, und wobei der Durchschnitt der Werte V der Eigenschaft oder Ausbeute von den mindestens 2 Standards $V_c$ ist.

3. Verfahren nach Anspruch 1 oder 2, umfassend Vergleichen von Absorptionen $D_{ix}$ (oder einer Ableitung davon) mit Absorption $D_{im}$ oder einer Ableitung davon.

4. Verfahren nach einem der Ansprüche 1-3, wobei der Standard $S_{mc}$ derart ist, dass in Bezug auf das Material X und den oder jeden Standard $S_{mc}$, die nachstehende Funktion erfüllt wird

$$\frac{i_{xm}}{\Sigma D_{ix}} < \text{Versuchsfehler}$$

worin $i_{xm}$ der Näherungs-Index ist und durch $i^2(xm) = \Sigma(D_{ix}-D_{im})^2$ definiert ist und der Versuchsfehler bei der Ermittlung der Eigenschaft oder Ausbeute beim Standard ist, und in dem Fall, wenn mehr als ein Standard $S_{mc}$ die Funktion erfüllt, ergibt Mitteln der Eigenschaften oder Ausbeuten aus den Standards einen Wert $V_c$.

5. Verfahren nach Anspruch 4, wobei der Vergleich von Signalen (i) mit Signalen (ii) erfolgt, die für Absorptionen $D_{im}$ bei derselben Wellenlänge oder eine mathematische Funktion davon bezeichnend sind, von einem Standard $S_{mc}$ mit dem bekannten Wert $V_c$ der Eigenschaft oder Ausbeute, und Steuern des Verfahrens, um zu sichern, dass die in Anspruch 4 ausgewiesene Funktion erfüllt wird.

6. Verfahren nach Anspruch 4 oder 5, wobei der Näherungs-Index kleiner als der Minimal-Index im ist, der aus den Standards Sa, Sb, Sc ...... bestimmt wurde, durch (a) Berechnen für jedes Paar von Standards Sa/Sb, Sa/Sc den Wert von $i^2(a,b)$ usw., (b) in Beziehung setzen der Werte von $i^2(a,b)$ usw. mit den entsprechenden Differenzen EP (ab) bei Eigenschaften Pa, Pb usw., (c) Berechnen für jeden Wert L, für den $i^2(ab) \le L$ ist, den Mittelwert der entsprechenden Differenzen EPab, (d) Berechnen des Minimal-Index aus dem Wert des kleinsten Index $i^2(ab)$, wobei der mittlere EPab derselbe ist, wie ein Reproduzierbarkeitsstandard für die Eigenschaft.

7. Verfahren nach einem der vorangehenden Ansprüche, wobei das Verfahren in einem geschlossenen Regelsystem gesteuert wird, um die verfahrenstechnische Ausrüstung in Bezug auf ein Verfahren, für das das Material X ein Zuführungsmaterial darstellt, zu steuern.

8. Verfahren nach einem der Ansprüche 1-7, das das Bestimmen der Ausbeute von mindestens einem Produkt aus dem Verfahren aus den Absorptionen des Zuführungsmaterials zu dem Verfahren umfasst.

9. Verfahren nach Anspruch 8, das das Bestimmen der Ausbeute von mindestens einem Alken von 2-4 Kohlenstoffatomen, Methan und Koks (wie durch einen Verkokungs-Index ausgedrückt) umfasst.

10. Verfahren nach einem der vorangehenden Ansprüche, das Computer-gestützt ist.

**Revendications**

1. Procédé de commande d'un processus de vapocraquage, pour lequel un matériau X constitue une amenée afin de maintenir constante la valeur $V_c$ d'une propriété P de cette amenée, ou le rendement de ce processus, le procédé consistant à mesurer l'absorption $D_ix$ du matériau à plus d'une longueur d'onde dans la gamme de 600 à 2 600 nm, **caractérisé en ce qu'**il consiste aussi à comparer les signaux (i) indiquant les absorptions ou une fonction mathématique de celles-ci avec des signaux (ii) indiquant les absorptions $D_im$ aux mêmes longueurs d'onde ou une fonction mathématique de celles-ci pour au moins 2 standards $S_m$ pour lesquels la propriété ou le rendement a une valeur connue V, au moins un de ces standards $S_{mc}$ ayant une valeur $V_c$ pour la propriété ou le rendement, et à commander le processus de façon à garantir que le standard $S_{mc}$ ou les standards $S_{mc}$ sont le standard ou les standards ayant la valeur moyenne plus faible ou la plus faible de la différence absolue à chaque longueur d'onde i entre le signal du matériau et le signal du standard $S_m$.

2. Procédé selon la revendication 1, qui comprend la comparaison des signaux (i) du matériau avec des signaux (ii) des standards $S_m$, dont au moins 2 ont les valeurs moyennes les plus faibles des différences et la moyenne des valeurs V de la propriété ou du rendement de ces, aux moins 2, standards étant $V_c$.

3. Procédé selon la revendication 1 ou 2, comprenant la comparaison des absorptions $D_ix$ (ou une de leurs dérivées) avec l'absorption $D_im$ ou une de ses dérivées.

4. Procédé selon l'une quelconque des revendications 1 à 3, ans lequel le standard $S_{mc}$ est tel que, par rapport au matériau X et le ou chaque standard $S_{mc}$, la fonction ci-après est remplie

$$i_{xm} < \frac{\text{erreur expérimentale}}{\sum D_{ix}}$$

où $i_{xm}$ est l'indice de proximité et est défini par $i^2(xm) = \Sigma(D_{ix} - D_{im})^2$ et l'erreur expérimentale se produit en déterminant la propriété ou le rendement dans le standard et, dans le cas où plus d'un standard $S_{mc}$ répond à cette fonction, l'établissement de la moyenne des propriétés ou des rendements donne une valeur $V_c$.

5. Procédé selon la revendication 4, dans lequel la comparaison des signaux (i) s'effectue avec des signaux (ii) indiquant les absorptions $D_{im}$ à la même longueur d'onde ou une fonction mathématique de celles-ci, d'un standard $S_{mc}$ ayant la valeur connue $V_c$ de la propriété ou du rendement et commandant le processus pour garantir que la fonction indiquée par la revendication 4 est satisfaite.

6. Procédé selon la revendication 4 ou 5, dans lequel l'indice de proximité est inférieur à l'indice minimal $i_m$ qui a été déterminé à partir des standards Sa, Sb, Sc ... (a) en calculant pour chaque paire de standards Sa/Sb, Sa/Sc la valeur de $i^2(a, b)$ etc., (b) en établissant la relation des valeurs de $i^2(a, b)$ etc. avec les différences correspondantes EP (ab) dans les propriétés Pa, Pb, etc., (c) en calculant pour chaque valeur L pour laquelle $i^2(ab)$ est $\leq L$, la moyenne des différences correspondantes EPab, (d) en calculant l'indice minimal à partir de la valeur de l'indice minimal $i^2$ (ab), où la moyenne EPab est la même que le standard de reproductibilité pour la propriété.

7. Procédé selon l'une quelconque des revendications précédentes, dans lequel le processus est commandé dans un système de commande en circuit fermé afin de commander le matériel de traitement par rapport à un processus pour lequel le matériau X est une amenée.

8. Procédé selon l'une quelconque des revendications 1 à 7, qui comprend l'étape consistant à déterminer, à partir des absorptions de l'amenée au processus, le rendement d'au moins un produit du processus.

9. Procédé selon la revendication 8, qui comprend l'étape consistant à déterminer le rendement d'au moins un parmi les alcènes en $C_2$-$C_4$, le méthane et le coke (exprimé par l'indice d'aptitude à la cokéfaction).

10. Procédé selon l'une quelconque des revendications précédentes, qui est mis en oeuvre au moyen d'un ordinateur.

## FIG.1

## FIG.2